# EUROPEAN PATENT APPLICATION

(11) **EP 2 351 508 A1**
(43) Date of publication of application: **03.08.2011**
(21) Application number: 08878428.5
(22) Date of filing: 28.11.2008
(51) Int. Cl.: A61B 1/00

(54) **LIVING BODY OBSERVATION APPARATUS**

(71) Applicant: OLYMPUS CORPORATION, Shibuya-ku Tokyo 151-0072 (JP)
(72) Inventor: KAWANO, Yoshihiro, Tokyo 151-0072 (JP)
(74) Representative: Schicker, Silvia
(86) International application number: PCT/JP2008/071651
(87) International publication number: WO 2010/061471

(57) **Abstract**

Tumor tissue or the like that exists at a deep portion of a living organism far from an internal wall of a body cavity is observed. A biological observation apparatus (1) is provided, which includes an inserted portion (2) that has a small-diameter distal end portion (2a) to be inserted into a body of a specimen (A), such as a mammal or the like, and that emits straight-line laser light (L) in a radial direction from the distal end portion (2a); and an image capturing portion (17) that is disposed outside of the body of the specimen (A) and that captures fluorescence generated from the specimen (A) due to the laser light (L) emitted from the inserted portion (2), wherein the inserted portion (2) is provided with a scanner (8) that scans the laser light (L) in a circumferential direction.

## Description

### Technical Field

The present invention relates to a biological observation apparatus.

### Background Art

In the related art, there is a known endoscope apparatus that is provided with an inserted portion that is inserted into a body cavity, that emits excitation light from a distal end of the inserted portion toward an internal wall of the body cavity, and that detects fluorescence generated by exciting fluorescent substances that exist in the internal wall of the body cavity by collecting the fluorescence with an objective lens disposed inside the inserted portion (for example, see Patent Citation 1).

Patent Citation 1: Japanese Unexamined Patent Application, Publication No. 2007-125403.

### Disclosure of Invention

With the endoscope apparatus disclosed in Patent Citation 1, however, because excitation light is emitted from a distal end of an inserted portion inserted into a body cavity and an image is formed by detecting fluorescence generated from a very limited region near an internal wall of the body cavity, there is a problem in that it is difficult to observe a deep portion of a living organism far from the internal wall of the body cavity.
The present invention has been conceived in light of the above-described circumstances, and an object thereof is to provide a biological observation apparatus that is capable of observing tumor tissue or the like that exists at a deep portion of a living organism far from an internal wall of a body cavity.

In order to achieve the above-described object, the present invention employs the following solutions.
The present invention provides a biological observation apparatus that includes an inserted portion that has a small-diameter distal end portion to be inserted into a body of a specimen, such as a mammal or the like, and that emits straight-line laser light in a radial direction from the distal end portion; and an image capturing portion that is disposed outside of the body of the specimen and that captures fluorescence generated in the specimen due to the laser light emitted from the inserted portion, wherein the inserted portion is provided with a scanner that scans the laser light in a circumferential direction.

With the present invention, the small-diameter distal end portion is inserted into the body of the specimen, such as a mammal or the like, and the straight-line laser light is emitted from the distal end of the inserted portion to excite fluorescent substances that exist in the path of the laser light, thereby generating fluorescence. The generated fluorescence passes through the specimen and is captured by the image capturing portion disposed outside the body. A fluorescence image acquired at this time by the image-capturing forms a one-dimensional straight-line fluorescence image that follows the laser light path.

Here, the image capturing portion is operated while scanning the laser light in the circumferential direction by operating the scanner. By doing so, a one-dimensional straight-line fluorescence image is acquired when the laser light is located at each position in the circumferential direction. Therefore, by combining these straight-line fluorescence images, a two-dimensional fluorescence image can be acquired.

By disposing the image capturing portion outside the body of the specimen, the optical system is not restricted; therefore, it is possible to employ an objective lens with a large numerical aperture or a high-sensitivity image capturing element. Accordingly, it also is possible to capture fluorescence generated by fluorescent substances that exist at a position relatively far from the distal end portion of the inserted portion which emits the laser light. In other words, by performing observation after administering a fluorescent agent that specifically accumulates in tumor tissue such as cancer or the like, it is possible to observe the tumor tissue or the like that exists at a deep portion of the specimen far from an internal wall of the body cavity.

With the above-described invention, the scanner may be provided with a mirror that is oscillated to and fro and an angle sensor that detects an angle of the mirror.
By doing so, a mirror angle is detected by operating the angle sensor, thereby making it possible to identify a laser-light scanning position of the scanner. As a result, it becomes possible to confirm relative relationships among the individual fluorescence images by storing the fluorescence images acquired by the image capturing portion in association with outputs of the angle sensor, thereby making it possible to confirm fluorescence generation sites afterwards.

In addition, with the above-described invention, an optical axis of the image capturing portion and a laser light scanning plane of the scanner may be arranged so as to intersect with each other.
By doing so, the straight-line fluorescence image can be observed from a direction that intersects with the line, thereby making it possible to more clearly observe the fluorescence generation sites.

Furthermore, the above-described invention may further include observation angle adjusting means for adjusting a relative angle of the optical axis of the image capturing portion with respect to the laser light scanning plane of the scanner.
By doing so, a relative angle between the optical axis of the image capturing portion and the laser light scanning plane is adjusted by operating the observation angle adjusting means, thereby making it possible to observe a tomogram of the specimen from an easy-to-view direction.

With the present invention, an advantage is afforded in that it is possible to observe tumor tissue or the like that exists at a deep portion of a living organism far from an internal wall of a body cavity.

### Brief Description of Drawings

[Fig. 1] Fig. 1 is an overall configuration diagram showing a biological observation apparatus according to an embodiment of the present invention.
[Fig. 2] Fig. 2 is a perspective view showing an illumination optical system of the biological observation apparatus in Fig. 1.

### Explanation of Reference:

A: specimen
L: laser light
P: scanning plane
1: biological observation apparatus
2: inserted portion
2a: distal end portion
8: scanner
13: mirror
14: motor (angle sensor)
17: CCD (image capturing portion)

### Best Mode for Carrying Out the Invention

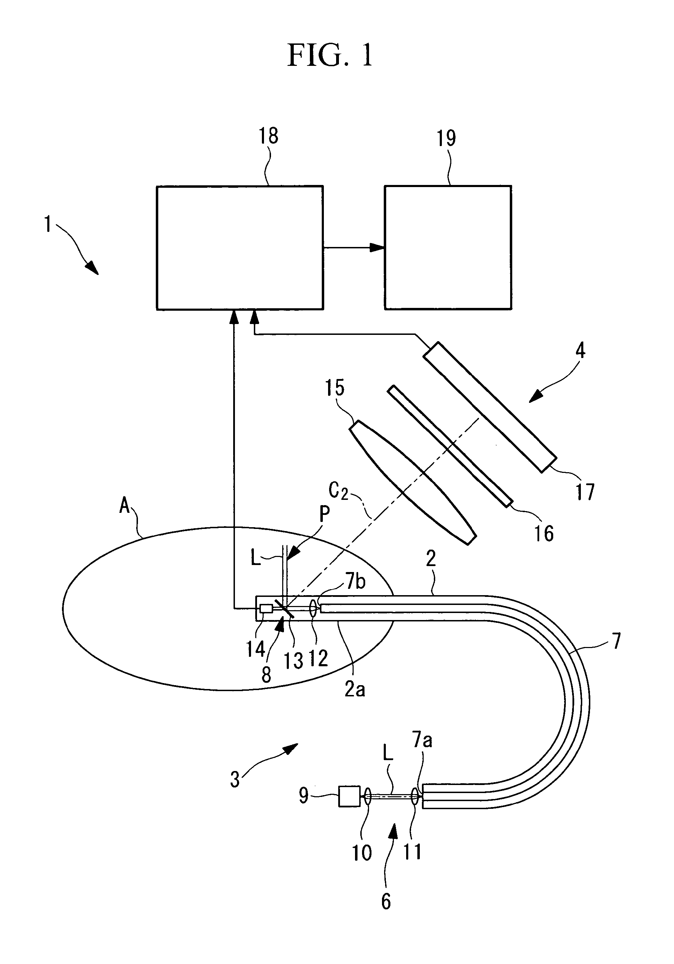

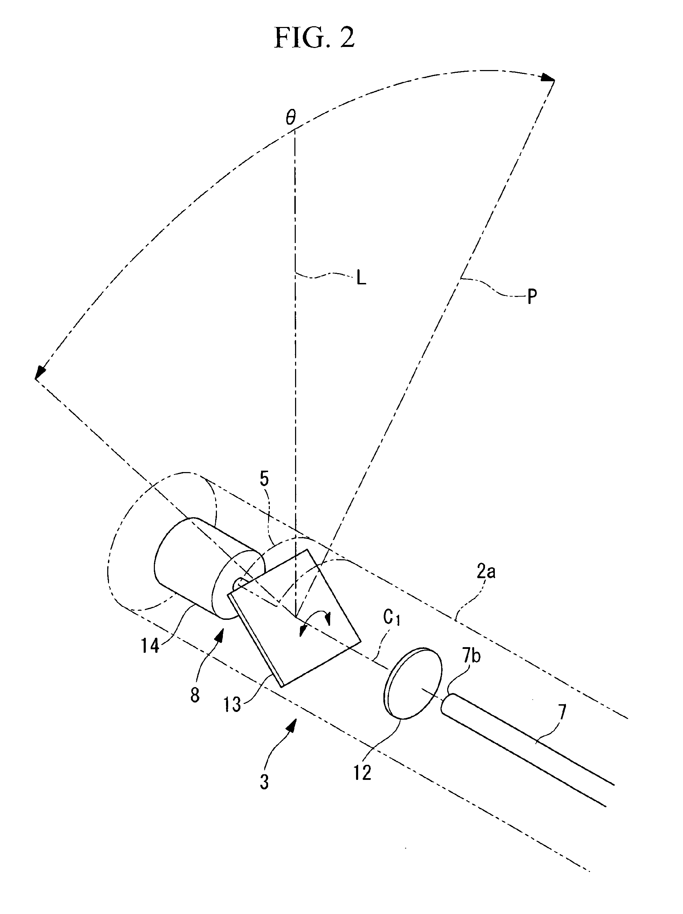

A biological observation apparatus 1 according to an embodiment of the present invention will be described below with reference to Figs. 1 and 2.
The biological observation apparatus 1 according to this embodiment is an apparatus for which a mammalian living organism, such as a mouse or the like, is used as a specimen A to observe tumor tissue or the like, such as cancer, etc., that exists inside it.

As shown in Fig. 1, the biological observation apparatus 1 according to this embodiment is provided with an inserted portion 2 that is inserted into the interior of the specimen A, such as a mammal or the like; an illumination optical system 3 that emits laser light L from a distal end of the inserted portion 2; and an observation optical system 4 for observing, outside the body, fluorescence generated due to the laser light emitted from the illumination optical system 3.

The inserted portion 2 is provided with a long, thin, flexible distal end portion 2a that can be inserted into the body cavity or the like in the specimen A. As shown in Fig. 2, a transparent window portion 5 from which laser light L coming from the illumination optical system 3 disposed thereinside is emitted radially outward by passing therethrough is provided at a side surface of the distal end portion 2a of the inserted portion 2. A transmitter (not shown) that transmits signals to the outside is provided at a distal end of the inserted portion 2, and, by receiving the signals outside the specimen A, an emitting position of the laser light L at the distal end of the inserted portion 2 can be identified.

The illumination optical system 3 is provided with a light source portion 6 that is disposed at a proximal end of the inserted portion 2 to generate the laser light L; an optical fiber 7 that guides the laser light L from the light source portion 6 to the distal end of the inserted portion 2; and a scanner 8 that deflects the laser light L that has been guided in a longitudinal direction of the inserted portion 2 by the optical fiber 7 in a radial direction to be scanned over a predetermined angular range.

The light source portion 6 is provided with a laser light source 9 that generates the laser light L; a collimating lens 10 that converts the laser light L emitted from the laser light source 9 to a substantially collimated beam; and a coupling lens 11 that focuses the laser light L that has been converted to a substantially collimated beam by the collimating lens 10 to an entrance-end surface 7a at a proximal end of the optical fiber 7.
The scanner 8 is provided with a collimating lens 12 that converts the laser light L that has been guided by the optical fiber 7 and emitted from an exit-end surface 7b at the distal end portion 2a of the inserted portion 2 to a substantially collimated beam; a mirror 13 that deflects the laser light L that has been converted to a substantially collimated beam by the collimating lens 12; and a motor (angle sensor) 14, with a built-in an angle position detector, for rotating the mirror 13.

The mirror 13 is disposed in an inclined manner at an angle of 45° relative to an optical axis C₁ of the laser light L that has been converted to a substantially collimated beam by the collimating lens 12. By doing so, the laser light incident on the mirror 13 can be deflected at 90° to be emitted radially outward from the window portion 5. In addition, the motor 14 is disposed so that a rotating shaft thereof is aligned with the optical axis C₁ of the laser light L, thereby making it possible to rotate the mirror 13 to and fro over a predetermined angular range θ about the optical axis C₁ of the laser light L.
By doing so, the laser light L emitted radially outward from the inserted portion 2 is oscillated back and forth with the rotation of the mirror 13 so as to be scanned in a sector-like manner while penetrating the specimen A in a thickness direction thereof.

The observation optical system 4 has an optical axis C₂ arranged at a predetermined angle relative to a scanning plane P of the laser light L and is provided with an objective lens 15 that collects fluorescence generated inside the specimen A; an excitation-light cut filter 16 that removes the laser light L from the fluorescence collected by the objective lens 15; a CCD 17 that captures the fluorescence that has passed through the excitation-light cut filter 16; an image processer 18 that is connected to the CCD 17 and the motor 14 and that creates a two-dimensional fluorescence image by processing fluorescence image information acquired by the CCD 17; and a monitor 19 that displays the fluorescence image created by the image processor 18.
The objective lens 15 is provided with an angle adjusting portion (not shown) that adjusts a relative angle of the optical axis C₂ of the objective lens 15 with respect to the scanning plane P of the laser light L.

The operation of the thus-configured biological observation apparatus 1 according to this embodiment will be described below.
To observe the specimen A, such as a mammal or the like, using the biological observation apparatus 1 according to this embodiment, in a state in which a fluorescent agent that specifically accumulates in tumor tissue, such as cancer, etc., is administered to or injected into the specimen A, the inserted portion 2 is inserted into the specimen A from an opening formed by an incision made in a body cavity, abdomen, etc., and the illumination optical system 3 and the observation optical system 4 are operated.

The laser light L emitted from the laser light source 9 is focused at the entrance-end surface 7a of the optical fiber 7 via the collimating lens 10 and the coupling lens 11, is guided to the distal end portion 2a of the inserted portion 2 via the optical fiber 7, and is then emitted from the exit-end surface 7b. After being converted to a substantially collimated beam by the collimating lens 12, the laser light L emitted from the optical fiber 7 is reflected at the mirror 13 to be deflected at 90° and is emitted radially outward from the window portion 5 of the inserted portion 2.

Due to its directionality, the laser light L travels in a straight line, penetrating the specimen A in the thickness direction thereof, and excites fluorescent substances that exist in its path, thereby generating fluorescence. Because a fluorescent agent that specifically accumulates in tumor tissue, such as cancer or the like, is administered to the specimen A, when tumor tissue exists in the path along which the laser light L passes, the fluorescent agent accumulated in the tumor tissue is excited to generate fluorescence. The generated fluorescence is emitted outside the body of the specimen A while being scattered in the specimen A, is collected by the objective lens 15, and is captured by the CCD 17 after passing through the excitation-light cut filter 16.

Fluorescence image information acquired by the CCD 17 in a single image-capturing round is one-dimensional image information that follows the straight path of the laser light L. Because of this, in the image processor 8, each piece of fluorescence image information is stored in association with a rotational angle of the motor 14, and multiple pieces of fluorescence image information acquired by repeating image-capturing every time the motor 14 is rotated by a predetermined angle are combined. By doing so, a two-dimensional fluorescence image along the scanning plane P of the laser light L can be acquired by this combining operation and can be displayed on the monitor 19.

In this case, depending on the insertion position of the inserted portion 2, an angle formed by the scanning plane P of the laser light L and the optical axis C₂ of the objective lens 15 sometimes become shallow; therefore, in such a case, by operating the angle adjusting portion to set the relative angle between the scanning plane P of the laser light L and the optical axis C₂ of the objective lens to a predetermined angle or above, for example, 45° or greater, the acquired fluorescence image can be more easily viewed.

In this way, with the biological observation apparatus 1 according to this embodiment, fluorescence generated due to the laser light L with high directionality emitted outward from inside the specimen A is captured outside the body with the observation optical system 4; therefore, unlike a conventional endoscope in which fluorescence is collected in the long, thin inserted portion 2, there is no size restriction for the observation optical system 4 including the objective lens 15. Accordingly, an objective lens 15 with a relatively large diameter, thus having a large numerical aperture, can be used, and a high-sensitivity CCD 17 can be employed.
As a result, an advantage is afforded in that, even with a specimen A formed of scattering materials, tumor tissue or the like that exists at a deep location far from the distal end of the inserted portion 2 can be detected.

Note that, in this embodiment, a two-dimensional fluorescence image in a sector-shaped scanning plane P is acquired by rotationally driving the mirror 13; however, means for moving the mirror 13 in the longitudinal direction of the inserted portion 2 may additionally be provided. By doing so, a two-dimensional fluorescence image is acquired at each position while moving the mirror 13 in the longitudinal direction, thereby making it possible to acquire positional information of a fluorescent section. In addition, instead of moving the mirror 13, fluorescence images may be acquired while moving the entire inserted portion 2 in the longitudinal direction.

In addition, in this embodiment, a two-dimensional fluorescence image along a sector-shaped scanning plane P is acquired by rotating the mirror 13 over the predetermined angle θ by operating the motor 14; however, approximately circular fluorescence images may be acquired by rotating the mirror 13 around the entire circumference.

In addition, although an example in which the mirror 13 is driven by the motor 14 has been described, a galvanometer mirror whose swivel angle is changed by altering the applied voltage may be employed. In this case, it is preferable that an angle sensor that detects an angle of a reflecting surface of the galvanometer mirror be provided. As the angle sensor, for example, a sensor formed of a laser diode and a line sensor that detects detection laser light emitted from the diode and reflected at the reflection surface of the galvanometer mirror can be employed.

Furthermore, although a flexible inserted portion 2 is employed in this embodiment, a rigid inserted portion 2, such as a rigid scope, may be employed. In this case, the laser light L may be guided to the distal end of the inserted portion 2 without using the optical fiber 7.

In addition, although the CCD 17 is employed as an image capturing portion, a CMOS or PMT array may be employed instead.
Furthermore, a single PMT may be employed. In this case, because only a single piece of brightness information is acquired for each rotational angle position of the motor 14, although it is not possible to generate a two-dimensional image, the presence or absence of tumor tissue or the like, as well as the direction thereof, can easily be identified.

## Claims

1. A biological observation apparatus comprising:
an inserted portion that has a small-diameter distal end portion to be inserted into a body of a specimen, such as a mammal or the like, and that emits straight-line laser light in a radial direction from the distal end portion; and
an image capturing portion that is disposed outside of the body of the specimen and that captures fluorescence generated in the specimen due to the laser light emitted from the inserted portion,
wherein the inserted portion is provided with a scanner that scans the laser light in a circumferential direction.

2. A biological observation apparatus according to Claim 1, wherein the scanner is provided with a mirror that is oscillated to and fro and an angle sensor that detects an angle of the mirror.

3. A biological observation apparatus according to Claim 1 or 2, wherein an optical axis of the image capturing portion and a laser light scanning plane of the scanner are arranged so as to intersect with each other.

4. A biological observation apparatus according to Claim 3, further comprising observation angle adjusting means for adjusting a relative angle of the optical axis of the image capturing portion with respect to the laser light scanning plane of the scanner.
